# EUROPEAN PATENT APPLICATION

(11) **EP 3 260 546 A1**
(43) Date of publication of application: **27.12.2017**
(21) Application number: 16305770.6
(22) Date of filing: 24.06.2016
(51) Int. Cl.: C12P 1/04, B09C 1/00, B09C 1/10, C02F 3/28, C02F 3/34, C12P 7/54, C12P 3/00

(54) **PROCESS FOR THE CO-CULTURE OF A BACTERIUM OF THE MESOTOGA LINEAGE AND AT LEAST ONE HYDROGENOTROPHIC SULFATE REDUCING BACTERIUM**

(71) Applicant: Institut de Recherche pour le Developpement (I.R.D.), 13572 Marseille Cedex 02 (FR); Institut National Sciences Appliquees Technologie, Tunis 1080 (TN)
(72) Inventor: OLLIVIER, Bernard, 13360 Roquevaire (FR); FARDEAU, Marie-Laure, 13170 Les Pennes Mirabeau (FR); BEN HANIA, Wajdi, 13009 Marseille (FR); HAMDI, Moktar, 2094 Ariana (TN); FADHLAOUI, Khaled, 2042 Tunis (TN)
(74) Representative: Bourgouin, André

(57) **Abstract**

The present invention concerns a process for the co-culture of an anaerobic mesophilic bacterium of the *Mesotoga* lineage with at least one hydrogenotrophic sulfate-reducing bacterium.

## Description

The present invention relates to a process for the co-culture of a bacterium of the *Mesotoga* lineage and at least one hydrogenotrophic sulfate-reducing bacterium.

For a long time, cultivated representatives within *Thermotogae* were known to be essentially thermophilic to hyperthermophilic. The situation has changed when 16S rRNA gene sequences have been reported in many mesothermic environments suggesting that a mesophilic lineage *(Mesotoga)* of *Thermotogae* exists (Nesbø *et al.,* 2006). The first mesophilic representative of *Thermotogae* (strain PhosAc3) was cultivated in 2011 (Ben Hania *et al.,* 2011), thereafter two *Mesotoga* species, *M. prima* MesG1.Ag.4.2^{T} (Nesbø *et al.,* 2012) and *M. infera* VNs100^{T} (Ben Hania *et al.,* 2013) were characterized. *Mesotoga* strain PhosAc3, from its side, was recently recognized as *a M. prima* strain (Ben Hania *et al.,* 2015). The three cultivated strains of *Mesotoga* exhibit a mesothermic range of growth temperature with optimum occurring between 37 °C and 45 °C and no growth occuring over 50 °C, thus demonstrating the existence of mesophilic species in *Thermotogae.*

To date, beside the genus *Mesotoga, Thermotogae* comprise 12 other genera including *Defluviitoga, Fervidobacterium, Geotoga, Oceanotoga, Petrotoga, Thermococcoides, Thermosipho, Thermotoga, Athalassotoga* and *Mesoaciditoga. Thermotogae* are usually considered as heterotrophic fermentative microorganisms able to use sugars, polysaccharides or complex organic substrates such as peptone and yeast extract.

Phosphogypsum (PG) is an industrial waste resulting from phosphate fertilizer production by treating apatite and phosphorite with sulfuric acid according to the following reaction:

Ca₃ (PO₄) + 3H₂SO₄ → 2H₃PO₄ + 3 CaSO₄

Around 5 tons of PG are generated per ton of phosphoric acid produced, depending on the phosphate rock source material (Azabou *et al.,* 2005, Nayak *et al.,* 2011, Tayibi *et al.,* 2009). The main components of PG are gypsum (CaSO₄ x 2H2O) and bassanite (CaSO₄ x 1/2H2O); sulfate ions accounts for approximately 50% (Marzenna *et al.,* 2010). PG also contain high concentration of hazardous impurities such as a heavy metals (Cd, Zn, Hg, Cu, Ni...) (May and Sweenly,1984; Luther *et al.* 1993; Azabou *et al.* 2005; El Zrelli *et al.* 2015) and radioactive elements (U²³⁸,Th²³⁰,Po²¹⁰,Ra²²⁶) (Borrego *et al.* 2007; Brunett *et al.* 1996; Rutherford *et al.* 1994). Several ways of valorization of phosphogypsum were developed; it is used in agriculture as fertilizer for the cultures of peanuts (Papastefanou *et al.,* 2006). The phosphogypsum was largely used in various applications of construction and building (De 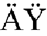 irmenci 2008, Singh et Garg, 2000). Other researchers tested the effect of the use of phosphogypsum on Portland cement. They proved that it can improve mechanical properties (Mehta et Brady, 1977; Kacimi *et al.,* 2006 ; Taher, 2007). Unfortunately, these solutions of treatment of phosphogypsum are not of economical relevance (A. Juszczak *et al.* 2002).

The anaerobic bioremediation process of PG by sulfate reducing bacteria has been of peculiar interest in recent years (Azabou *et al.* 2007; Dorota and Andrzej 2009).

Successful experiments have been undertaken to cultivate sulfate- reducing bacteria on PG in the presence of hydrogen, acetate, lactate, formate, propionate and butyrate as electron donors (Azabou *et al.* 2007). Despite significant results have been obtained with regard to heavy metals removal in these conditions, the use of expensive organic compounds (e.g. lactate) or dangerous gas (e.g. H2) as compared to cheap easily available substrates (e.g. sugars) is still an obstacle to the use of this biological process.

Such a process makes the cleanup of soil contaminated by phosphogypsum, expensive and dangerous.

The Inventors have surprisingly discovered that co-culturing *Mesotoga* species with hydrogenotrophic sulfate-reducing partners in the presence of a sulfur source, in particular in presence of sulfate or elemental sulfur, as terminal electron acceptors, significantly improved carbon source oxidation by the former. Thus, they demonstrate two major points regarding *Mesotoga* species: (i) their dependence on the presence of a sulfur source as terminal electron acceptor to use glucose, thus indicating that in contrast to all *Thermotogae* known so far, sugars are rather oxidized than fermented and (ii) the efficient syntrophic association with hydrogenotrophic sulfate-reducing partners as biological terminal electrons facilitating carbon source oxidation.

The Inventors have thus demonstrated an unusual prokaryotic metabolism within the *Thermotogae*, e.g. the obligate oxidation of carbohydrates (known as easily fermented by other members within the *Thermotogae* in the absence of any chemical or biological electron acceptor) thanks to a syntrophic association with a hydrogenotrophic sulfate-reducing partner and its possible ecological significance in nature.

The Inventors have furthermore surprisingly discovered that the syntrophic association between an anaerobic mesophilic bacterium from *Mesotoga* lineage and a hydrogenotrophic sulfate-reducing bacterium was able to use sulfate originating from phosphogypsum, in the presence of a carbon source, such as sugars (hexoses and pentoses), producing acetate and sulfide.

Thus, they have discovered a process for the cleanup of soils contaminated by heavy metals, metalloids and radionucleids originating from phosphogypsum, using a cheap easily available substratum.

The present invention concerns a process for the co-culture of an anaerobic mesophilic bacterium of the *Mesotoga* lineage with at least one hydrogenotrophic sulfate-reducing bacterium.

The term "hydrogenotrophic" refers to a microorganism which oxidizes hydrogen as energy source in the presence of a terminal electron acceptor (e.g. sulfate, carbon dioxide etc.) .

The term "sulfate-reducing" refers to a microorganism which is able to obtain energy by oxidizing organic compounds or molecular hydrogen (H₂) while reducing sulfate (SO₄²⁻) to hydrogen sulfide (H₂S). In a sense, these organisms perform a sulfate respiration under anaerobic conditions.

In a particular embodiment, the present invention relates to a process for the co-culture of an anaerobic mesophilic bacterium of the *Mesotoga* lineage with at least one hydrogenotrophic sulfate-reducing bacterium, wherein the said co-culture is performed in a culture medium comprising at least one energy source and at least one sulfur source.

In a particular embodiment, the present invention relates to a process for the co-culture of an anaerobic mesophilic bacterium of the *Mesotoga* lineage with at least one hydrogenotrophic sulfate-reducing bacterium, wherein the said energy source is a carbon source.

In a particular embodiment, the present invention relates to a process for the co-culture of an anaerobic mesophilic bacterium of the *Mesotoga* lineage with at least one hydrogenotrophic sulfate-reducing bacterium, wherein the said co-culture is performed in a culture medium comprising at least one carbon source and at least one sulfur source.

The term "energy source" refers to any mineral (e.g. hydrogen) or organic compound (e.g. carbohydrates, amino acids, fatty acids, etc.) that is used to deliver energy (ATP) during the process of catabolism.

The term "carbon source" refers to any carbon containing molecules (carbohydrates, amino acids, fatty acids, carbon dioxide) used by an organism for its growth and subsequent synthesis of organic molecules during anabolism.

The term « sulfur source » refers to any molecule under an oxidized form containing at least one sulfur atom, including elemental sulfur and sulfate.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sulfur source is sulfate and wherein the said at least one carbon source is a sugar.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sulfur source is elemental sulfur and wherein the said at least one carbon source is a sugar.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sulfur source is phosphogypsum containing sulfate.

In a particular embodiment, the present invention relates to a process for the co-culture of an anaerobic mesophilic bacterium of the *Mesotoga* lineage with at least one hydrogenotrophic sulfate-reducing bacterium, wherein the said anaerobic mesophilic bacterium of the *Mesotoga* lineage is the bacterial strain PhosAc3, corresponding to the bacterial strain deposited at Collection Nationale de Cultures de Microorganismes (CNCM) under n°I-4238, or mutant thereof.

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is chosen among the *Deltaproteobacteria* class.

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is chosen among the *Deltaproteobacteria* class, particularly among the *Desulfovibrionales* order and particularly among the *Desulfovibrio* genus, the *Desulfomicrobium* genus and the *Desulfocurvus* genus.

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is the species *Desulfovibrio vulgaris* of the *Desulfovibrio* genus, particularly the subspecies *Desulfovibrio vulgaris* subsp. *vulgaris.*

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is chosen among the *Clostridia* class.

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is chosen among the *Clostridia* class, particularly among the *Clostridiales* order and particularly among the *Desulfotomaculum* genus and the *Desulfosporosinus* genus.

In a particular embodiment, the present invention relates to a process for the co-culture of an anaerobic mesophilic bacterium of the *Mesotoga* lineage with at least one hydrogenotrophic sulfate-reducing bacterium, wherein the said hydrogenotrophic sulfate-reducing bacterium is the species *Desulfotomaculum ruminis* of the *Desulfotomaculum* genus.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sugar is a C6 sugar.

In a particular embodiment, the present invention relates to a process for the co-culture of an anaerobic mesophilic bacterium of the *Mesotoga* lineage with at least one hydrogenotrophic sulfate-reducing bacterium, wherein the said C6 sugar is fructose.

In a particular embodiment, the present invention relates to a process for the co-culture of an anaerobic mesophilic bacterium of the *Mesotoga* lineage with at least one hydrogenotrophic sulfate-reducing bacterium, wherein the said C6 sugar is glucose.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sugar is a C5 sugar.

In a particular embodiment, the present invention relates to a process for the co-culture of an anaerobic mesophilic bacterium of the *Mesotoga* lineage with at least one hydrogenotrophic sulfate-reducing bacterium, wherein the said C5 sugar is xylose.

In a particular embodiment, the present invention relates to a process for the co-culture of an anaerobic mesophilic bacterium of the *Mesotoga* lineage with at least one hydrogenotrophic sulfate-reducing bacterium, wherein the said sugar is sucrose.

In a particular embodiment, the present invention relates to a process for the co-culture of an anaerobic mesophilic bacterium of the *Mesotoga* lineage with at least one hydrogenotrophic sulfate-reducing bacterium, wherein the said sugar is lactose.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sugar has a concentration from 2 mM to 50 mM, particularly from 10 mM to 20 mM.

In a particular embodiment, the present invention relates to a process for the co-culture of an anaerobic mesophilic bacterium of the *Mesotoga* lineage with at least one hydrogenotrophic sulfate-reducing bacterium, wherein the said co-culture is carried out at a temperature ranging from 20 °C to 50 °C.

In a particular embodiment, the present invention relates to a process for the co-culture of an anaerobic mesophilic bacterium of the *Mesotoga* lineage with at least one hydrogenotrophic sulfate-reducing bacterium, wherein the said co-culture is carried out at the temperature of 37°C.

In a particular embodiment, the present invention relates to a process for the co-culture of an anaerobic mesophilic bacterium of the *Mesotoga* lineage with at least one hydrogenotrophic sulfate-reducing bacterium, wherein the said at least one sulfur source is sulfate at a concentration ranging from 2 to 40 mM, particularly 20 mM.

In a particular embodiment, the present invention relates to a process for the co-culture of an anaerobic mesophilic bacterium of the *Mesotoga* lineage with at least one hydrogenotrophic sulfate-reducing bacterium, wherein the said anaerobic mesophilic bacterium is the bacterial strain PhosAC3 deposited at Collection Nationale de Cultures de Microorganismes (CNCM) CNCM under n°I-4238 and the said one hydrogenotrophic sulfate-reducing bacterium is *Desulfovibrio vulgaris,* said co-culture being performed in a culture medium containing sulfate and glucose.

In a particular embodiment, the present invention relates to a process for the co-culture of an anaerobic mesophilic bacterium of the *Mesotoga* lineage with at least one hydrogenotrophic sulfate-reducing bacterium, wherein the said anaerobic mesophilic bacterium is the bacterial strain PhosAC3 deposited at Collection Nationale de Cultures de Microorganismes (CNCM) CNCM under n°I-4238 and the said one hydrogenotrophic sulfate-reducing bacterium is *Desulfotomaculum ruminis,* said co-culture being performed in a culture medium containing sulfate and glucose.

The present invention also concerns a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar.

The present invention also concerns a method for the cleanup of sites/sediments contaminated by heavy metals, particularly contained in phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar.

According to the invention, heavy metals essentially include As, Cd, Co, Cr, Cu, Hg, Mn, Ni, Pb, Sn, Tl and Zn.

In a particular embodiment, the present invention relates to a method for the cleanup of sites/sediments contaminated by Zn contained in phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage with a hydrogenotrophic sulfate-reducing bacterium, preferably cultured according to the process described above, in presence of at least one sugar.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said anaerobic mesophilic bacterium of the *Mesotoga* lineage is the bacterial strain PhosAC3 deposited at Collection Nationale de Cultures de Microorganismes (CNCM) CNCM under n°I-4238.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said hydrogenotrophic sulfate-reducing bacterium is chosen among the *Deltaproteobacteria* class.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said hydrogenotrophic sulfate-reducing bacterium is chosen among the *Deltaproteobacteria* class, particularly among the *Desulfovibrionales* order and particularly among the *Desulfovibrio* genus, the *Desulfomicrobium* genus and the *Desulfocurvus* genus.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said hydrogenotrophic sulfate-reducing bacterium is the species *Desulfovibrio vulgaris* of the *Desulfovibrio* genus, particularly the subspecies *Desulfovibrio vulgaris* subsp. *vulgaris.*

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said hydrogenotrophic sulfate-reducing bacterium is chosen among the *Clostridia* class, particularly among the *Clostridiales* order and particularly among the *Desulfotomaculum* genus and the *Desulfosporosinus* genus.

In a particular embodiment, the present invention relates to a method for the cleanup of sites/sediments contaminated by phosphogypsum, comprising treating the contaminated sites/sediments with a co-culture of anaerobic mesophilic bacterium of the *Mesotoga* lineage, with a hydrogenotrophic sulfate-reducing bacterium, in presence of at least one sugar, wherein the said hydrogenotrophic sulfate-reducing bacterium is the species *Desulfotomaculum ruminis* of the *Desulfotomaculum* genus.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said at least one sugar is a C6 sugar.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said C6 sugar is glucose or fructose.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said at least one sugar is a C5 sugar.

In a particular embodiment, the present invention relates to a method for the cleanup of sites/sediments contaminated by phosphogypsum, comprising treating the contaminated sites/sediments with a co-culture of anaerobic mesophilic bacterium of the *Mesotoga* lineage, with a hydrogenotrophic sulfate-reducing bacterium, in presence of at least one sugar, wherein the said C5 sugar is xylose.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said at least one sugar is a sucrose or lactose.

In a particular embodiment, the present invention relates to a method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar, wherein the said at least one sugar is glucose and wherein the said anaerobic mesophilic bacterium of the *Mesotoga* lineage is the bacterial strain PhosAC3 deposited at Collection Nationale de Cultures de Microorganismes (CNCM) CNCM under n°I-4238 and the said hydrogenotrophic sulfate-reducing bacterium is *Desulfovibrio vulgaris.*

### Material and Methods

### Media and culture conditions

*Mesotoga prima* strain PhosAc3 corresponds to the bacterial strain CNCM I-4238 deposited at Collection Nationale de Cultures de Microorganismes on November 3, 2009.

*M. prima* strain PhosAc3 was grown with glucose (20mM) at 37°C in a basal medium (BM) containing per liter of distilled water 0.3 g KH₂PO₄; 0.3 g K₂HPO₄; 1.0 g NH₄Cl; 2.0 g NaCl; 0.1 g KCl; 0.1 g CaCl₂.2H₂O; 0.5 g MgCl₂.6H₂O; 1 g yeast extract; 0.5 g cysteine-HCl; 0,16 g sodium acetate ; 1 mL Widdel trace element solution (Widdel and Pfennig, 1984) and 1 mL resazurin 0.1 %. When specified, 10 grams of elemental sulfur (S°) were added per liter of medium.
Before culture inoculation, 0.2 mL of 10% (w/v) NaHCO₃, 0.1 mL of 2% (w/v) Na₂S-9H₂O and glucose, were injected from sterile stock solutions in the culture medium.

The sulfate-reducing bacteria *Desulfovibrio vulgaris* subsp. vulgaris and *Desulfotomaculum gibsoniae* were grown in the same culture medium as described for *M. prima* strain PhosAc3 with glucose being replaced by hydrogen (H₂-CO₂; 80:20 v/v) as electron donor and elemental sulfur by sodium sulfate (Na₂SO₄) 4 g/L, as electron acceptor.

Co-culture were performed in the same medium as for culturing M. prima PhosAc3, except that elemental sulfur was replaced by sodium sulfate (4 g/L).

Control culture of *M. prima* strain PhosAc3 was grown in the same culture medium without elemental sulfur and without hydrogenotrophic sulfate-reducing bacterium.

Cultures and co-cultures (obtained by mixing exponential growth cultures of *Mesotoga* strain PhosAC3 and sulfate-reducing bacteria 10% v/v each) were performed under anaerobic conditions in Hungate tubes or in large volume flasks inoculated with 10 % of growth exponential phase culture or co-culture and incubated at 37°C. Growth was monitored by measurement of the OD at 600 nm of the culture directly in the Hungate tube.

For co-culture in the presence of phosphogypsum as electron acceptor (sulfate source), cultures were performed under anaerobic conditions at 37°C in penicillin bottles.
The studied sample of PG was collected from a phosphogypsum stock at Sfax, Tunisia. The phosphogypsum samples were well homogenized by means of shaking.

### Analytical methods

Soluble sulfides were quantified according to the Cord-Ruwish method (1985) by using a Shimadzu UV-160A spectrophotometer (Shimadzu Co., Kyoto, Japan). In an acid medium, sulfides were converted into colloidal solution of copper sulfide CuS by reaction with copper sulfate. The copper sulfide was determined spectrophotometrically at 480 nm. One hundred µL of the sample was added to 4 mL of an assay solution (50 mM HCl, 5 mM copper sulfate). The reaction mixture was vortexed before reading the optical density. The determination of the sulfide concentration was done by using a calibration curve made with Na₂S-9H₂O. To determine the total sulfides, partitioned between the gas phase and liquid of the culture, the equilibrium was shifted to the liquid phase by raising the pH (up to 12) by addition of aliquot of 10 M NaOH.

Sugars (lactose, glucose, fructose, sucrose), organic acids (acetate, lactate, propionate, butyrate ...) and ethanol concentrations are carried out by high performance liquid chromatography (HPLC) as described in Fardeau *et al.,* (1997) by using a differential refractometer detector (Shimadzu RID 6 A, Japan) connected to a computer running WINILAB III software (Perichrom, France). Briefly, 1 mL samples were withdrawn from the culture at regular time interval. Samples were then centrifuged for 5 minutes at 14,500 rpm and 20 µL of the supernatant was loaded onto an Animex HPX-87H column (Biorad) set at 35°C and eluted at 0.5mL min⁻¹ with H₂SO₄ solution (0.75 mM). Product concentrations were determined by using a differential refractometer detector (Shimadzu RID 6 A, Japan) connected to a computer running WINILAB III software (Perichrom, France).

For hydrogen quantification, one mL of culture headspace sample was injected into a TCD-GC system (Shimadzu 8A, Japan) equipped with a concentric CTR1 column (Alltech, USA), connected to a computer running WINILAB III software (Perichrom, France) (Fardeau *et al.,* 1997). Unless otherwise indicated, analytical measures were performed on duplicate culture tubes or bottles.

Soluble sulfides were quantified according to the Cord-Ruwish method (1985) by using a Shimadzu UV-160A spectrophotometer (Shimadzu Co., Kyoto, Japan).

Zinc concentrations present in the soluble fraction were determined by atomic absorption spectrometry (Spectra AA 220 FS, Varian) as described by Bouain et al. (2014) (Bouain N., Kisko M., Rouached A., Dauzat M., Lacombe B., Belgaroui N., Ghnaya T., Davidian J-C., Berthomieu P., Abdelly C., Rouached H., 2014. Phosphate/Zinc Interaction Analysis in Two Lettuce varieties Reveals Contrasting Effects on Biomass, Photosynthesis, and Dynamics of Pi Transport. BioMed Research International, Volume 2014, article ID 548254).

### FIGURES

**Figure 1****:** (A) Glucose consumption in either pure culture of *M. prima* in the absence (black square) or presence (white circle) of elemental sulfur or co-culture with *Desulfovibrio vulgaris* subsp. *vulgaris* (white triangle) over the time.
   (B) Acetate production in either pure culture of *M. prima* in the absence (black square) or presence (white circle) of elemental sulfur or co-culture with *Desulfovibrio vulgaris* subsp. *vulgaris* (white triangle) over time.
**Figure 2****:** (A) evolution of the growth rate of the co-culture in function of the number of subculturing. (B): Growth curve of *M. prima* (■) and *D. vulgaris* (A) in pure culture or in co-culture (●) in glucose/sulfate medium.
**Figure 3****:** Concentration of glucose (A), of acetate production (B), and of sulfide production (C) by pure culture of *Mesotoga prima* strain PhosAc3 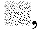 , pure culture of *Desulfovibrio vulgaris* subsp. *vulgaris* 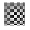 and co-culture *Mesotoga-Desulfovibrio* ■ in the presence of different concentrations of phosphogypsum after three weeks of incubation.
**Figure 4****:** Sugar consumption (A), acetate production (B), and sulfide production (C) after 3 weeks of incubationby pure culture of *Mesotoga prima* strain PhosAc3 (MESO), pure culture of *Desulfovibrio vulgaris* subsp. *vulgaris* (DVV) and co-culture *Mesotoga*-*Desulfovibrio*. Negative control (without carbon source), 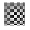 glucose, ■ fructose, ■sucrose.
**Figure 5****:** Time evolution of sugar consumption (A), acetate (B) and total sulfide (C) production by co-culture *(Mesotoga-Desulfovibrio)* for negative control 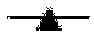 (without carbon source), fructose 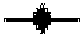 and glucose 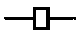 as carbon source.

### EXAMPLES

### I- Growth of Mesotoga prima PhosAC3 in pure culture and in co-culture with sulfate-reducing bacteria

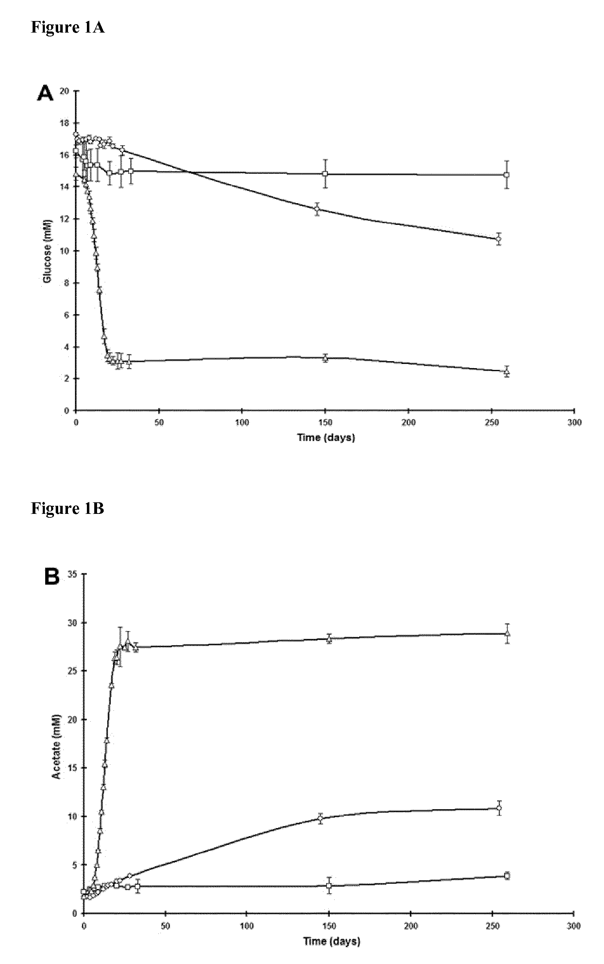

When *M. prima* PhosAc3 was cultured with glucose, only a slight glucose consumption was observed even after 250 days of incubation at 37°C only in the presence of yeast extract **(****Figure 1A****).** When elemental sulfur was added in the medium, a slow linear degradation of glucose was measured with 6.62 ± (0.19) mM of the glucose consumed (around 33%) after 250 days of incubation **(****Figure 1A****, table 1).** Accordingly, only a slight acetate production was measured in the absence of elemental sulfur while 10 mM acetate was produced in its presence after 250 days. The end-products of glucose metabolism detected were only acetate **(****Figure 1B****),** CO₂ and sulfide **(Table 1).** Surprisingly, only trace amounts of hydrogen (less than 1 µM) were detected in the gas phase during glucose consumption whatever the presence or the absence of elemental sulfur.

These data clearly showed that *M. prima* PhosAc3 was unable to ferment glucose while in the presence of an external electron acceptor (elemental sulfur), it was able to oxidize glucose although with a low efficiency as only around 33% of the initial glucose was consumed after 250 days of incubation. The slight glucose consumption in the absence of elemental sulfur was probably due to the presence of an electron acceptor available in yeast extract since hydrogen was detected in the gas phase at concentration as low as 1µM (limit of detection by gas chromatography).

**Table 1 : End-product quantification of the single and co-cultures of M. prima PhosAc3 after 250 days of incubation.**

| | H₂S produced (mM) | Acetate produced (mM) | Glucose consumed (mM) |
|---|---|---|---|
| *M. prima* | 1.05 ± 0.25 | 1.67 ± 0.21 | 1.50 ± 0.92 |
| *M. prima* + elemental sulfur | 24.40 ± 0.30 | 9.21 ± 0.13 | 6.57 ± 0.19 |
| *M. prima* + *Desulfovibrio vulgaris* | 9.70 ± 0.85 | 27.13 ± 0.45 | 12.39 ± 0.33 |

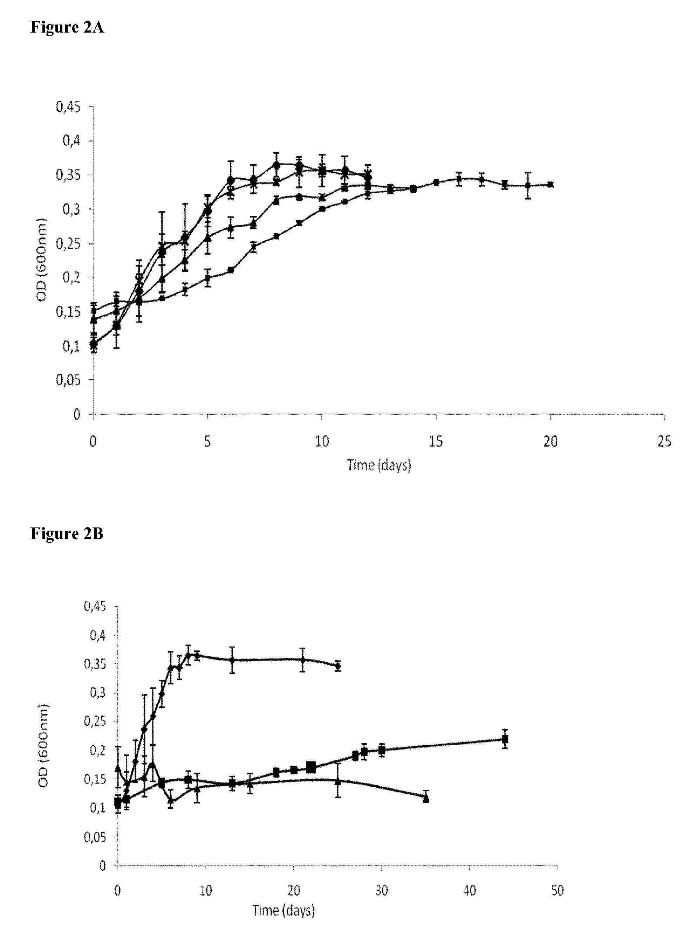

*Desulfovibrio vulgaris* subsp. *vulgaris* was used to test the syntrophic association with *M. prima* PhosAc3. Sulfate-reducing bacteria are anaerobic prokaryotes which gain energy for biosynthesis and growth by coupling oxidation of organic compounds or molecular hydrogen to reduction of sulfate to sulfide. When either *M. prima* PhosAC3 or *Desulfovibrio vulgaris* subsp. *vulgaris* alone was cultured in a glucose/sulfate medium, no growth was observed **(****Figure 2B****).** However, when the two bacteria were co-cultured, growth occurred as evidenced by a substantial increase in the optical density at 580 nm **(****Figure 2A and 2B****)** and in sulphide and acetate produced **(Table 1).** 80% of the initial glucose was consumed (-12 mM) with the concomitant acetate production of ∼ 25 mM after only 20 days of incubation **(****Figures 1A-1B****).** After 20 days, glucose consumption and acetate production only slighty progressed to give -13 mM glucose consumed and ∼28 mM acetate produced after 250 days of incubation **(****Figures 1A-1B****, Table 1).** At this time, about 10 mM sulfide had been produced **(Table 1).**

Similar results were obtained when *M prima* PhosAc3 was co-cultured with another sulfate-reducing bacterium (SRB) *Desulfotomaculum gibsonia* (data not shown). In the same way, *M. infera* VNs100^{T}, which was also not able to grow on glucose in pure culture in the absence of elemental sulfur as terminal electron acceptor, grew well when it was co-cultured with *Desulfovibrio vulgaris* subsp. *vulgaris* (data not shown).

### II - Co-culture in the presence of phosphogypsum as electrons acceptor

Experiments using basal medium and phosphogypsum (PG) as sulfate source have been performed with the aim to produce sulfide and also remove heavy metals from the waste of the industry producing phosphoric acid. The above experiments have demonstrated that a syntrophic association between *Mesotoga prima* strain PhosAC3 and *D. vulgaris* to oxidize glucose was successful. Thus, this co-culture was tested using economically advantageous substrates such as sugars, taking into account that the traditionally used substrates for treating PG are much more expensive (e.g lactate) and even dangerous (e.g. hydrogen) (Azabou *et al.* 2007) through industrial application. Neither *Mesotoga prima* strain PhosAC3 nor *D. vulgaris* were able to grow alone in the culture medium designed. In contrast, as demonstrated previously, when both bacteria *Mesotoga prima* strain PhosAC3 and *Desulfovibrio vulgaris* subsp. *vulgaris* were co-cultivated, growth was obtained in the basal medium containing on glucose (20 mM) in the presence of phosphogypsum (10g/l) after three weeks of incubation at 37°C.

Acetate and sulfide were the main end-products of glucose oxidation by this co-culture as reported earlier. They result from a syntrophic association between *Mesotoga prima* strain PhosAc3 and *D. vulgaris* subsp. *vulgaris* where the former acts as the glucose oxidizing bacterium, while the latter acts as hydrogen and/or formate scavenger since none of these compounds accumulate in the gas (H₂) or the liquid (formate) phase.

The turbidity caused by the presence of phosphogypsum in the culture medium prevented the measurement of the bacterial growth by the optical density. Therefore the proportioning by HPLC of the consumption of glucose and production of acetate by *Mesotoga prima* strain PhosAc3, and sulfide production by *D. vulgaris* were used.

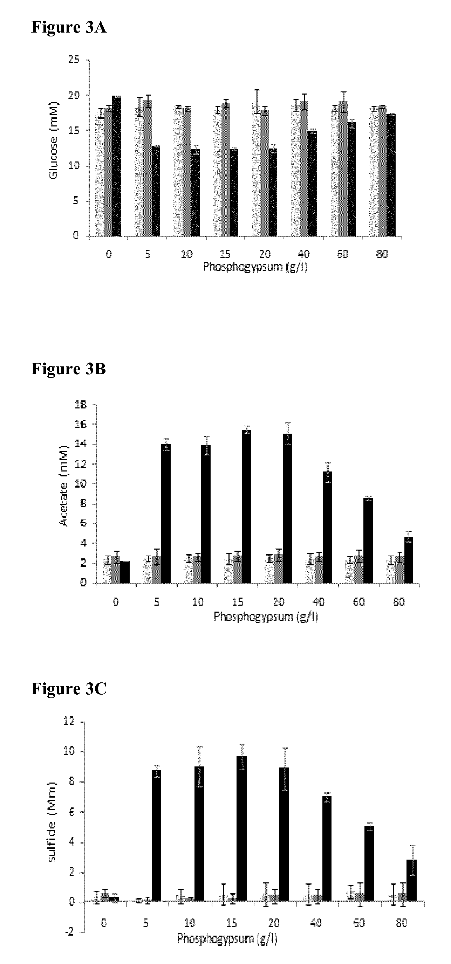

The syntrophic association between *Mesotoga Prima* strain phosAc3 and *Desulfovibrio vulgaris* subsp. *vulgaris* used the sulfate containing in the phosphogypsum as electron acceptor as confirmed by sulfide production **(****Figure 3C****).**

### II- 1. Optimal concentration of phosphogypsum as sulfate source

The determination of the optimal concentration of phosphogypsum for the growth of pure culture of *Mesotoga prima* strain PhosAC3, *Desulfovibrio vulgaris* subsp. *vulgaris* and co-culture *Mesotoga-Desulfovibrio* was carried out in the presence of 20 mM of glucose in the culture medium. Concentration from 0 to 80 gram per liter of phosphogypsum in the culture medium was tested. The bacterial growth was estimatedby measuring glucose consumption and acetate production on the one hand and the measuring ofsulfide production on the other hand. After three weeks of co-culture incubation, the optimum phosphogypsum concentration was obtained from 5 to 20 g/l followed by 40 g/l **(****Figure 3A**).

Starting from an initial concentration of glucose (∼20 mM), **Figure 3A** shows a consumption of 7,47 mM +/- 0,24 of glucose accompanied by a production of 12,44 mM +/- 0,77 of acetate by the co-culture in the presence of 5, 10 and 20 g/l phosphogypsum, **(****Figure 3B****).** The consumption of glucose by the co-culture observed in the presence of 80 g/l of phosphogypsum was only 2,5 mM +/- 0,17 with a production of 4,7 mM +/- 0,51 of acetate **(****Figure 3B)** and 3 mM +/- 0,78 of sulfides **(****Figure 3C****).**

No bacterial growth was observed in the absence of phosphogypsum by the co-culture. No bacterial growth was detected for two pure cultures tested (*Mesotoga* alone and *Desulfovibrio* alone) in the presence of different concentrations of phosphogypsum.

In this experiment, when grown on sugars, the tested co-culture tolerated up to 80 g/l of phosphogypsum and optimal growth was observed at concentrations ranging from 5 to 20 g/l of phosphogypsum. Maximum sulfide production was 15 mM. Co-culture growth decreased with concentration of phosphogypsum higher than 20 g/l due possibly to inhibition caused by the toxic effect of sulfide or heavy metals/metalloids contained in phosphogypsum.

### II- 2. Sugar utilization by the pure culture and the co-culture

20 mM of glucose, fructose and sucrose were tested in order to determine the preferred sugar to be used by the co-culture *Mesotoga - Desulfovibrio* in the presence of 15 g/l of phosphogypsum.

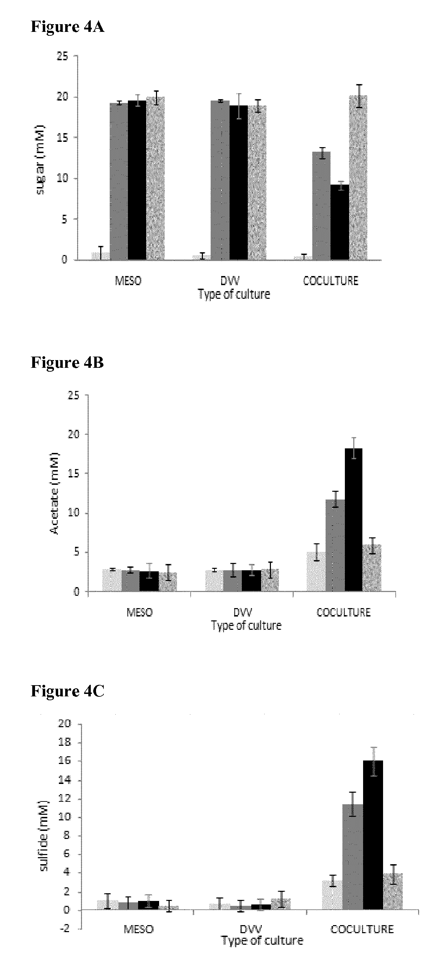

**Figure 4A** shows that fructose was the preferred carbon/energy source to be used by the co-culture (11mM +/- 0,77) which yielded the highest acetate and sulfide production 16 +/- 1,5 and 18,32 +/- 1,37 Mm respectively, followed by glucose with 7,12 +/- 0,76 mM of glucose consumed for a production of 11,84 +/- 1,03 mM of acetate **(****Figure 4B****)** and 11,42 +/- 1,31 mM of sulfides **(****Figure 4C****),** respectively.

Slight growth was obtained by the co-culture in the absence of sugar thus demonstrating that the co-culture can use yeast extract (1 g/l) present in the culture medium.

Neither *Mesotoga* strain PhosAC3 nor *Desulfovibrio vulgaris* subsp. *vulgaris* were shown to use sugars in pure cultures **(****Figure 4A**).

This is the first time that a high sulfide production (15 and 19 mM of sulfide produced from glucose and fructose oxidation respectively) was obtained for phosphogypsum treatment using sugars as carbon source.

### II- 3. Kinetics of sugar consumption, acetate and sulfide production by the co-culture Mesotoga-Desulfovibrio

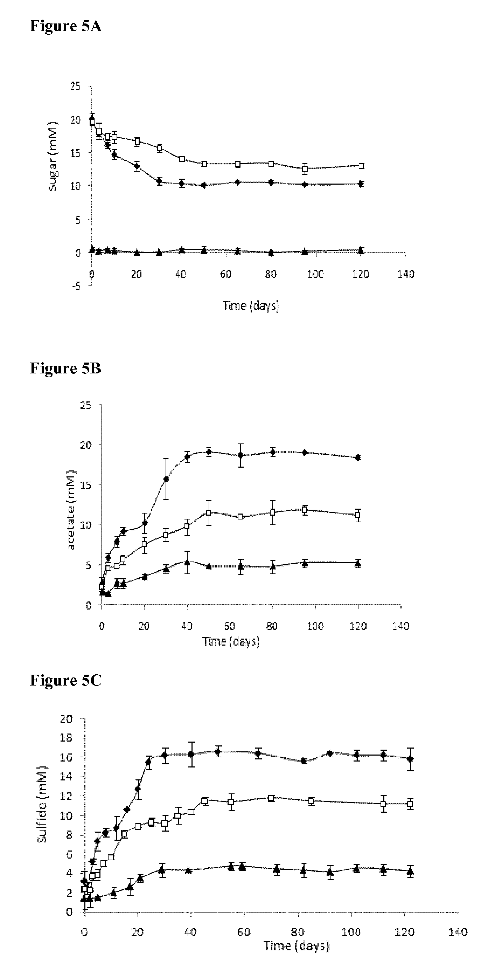

**Figure 5A** shows the evolution on time of glucose and fructose (20 mM) degradation by the co-culture, in the presence of 15 g/l of phosphogypsum as sulfate source. 30 days are essential to obtain maximum sulfide production of 16,2 +/- 0,92 mM **(****Figure 5C****)** which is clearly correlated to sugar consumption and acetate production (18,5 +/- 0,12 mM) **(****Figure 5A and 5B****),** in the presence of fructose.

In term of incubation (between 20 and 30 days incubation), similar results with the production of 11,5 mM +/- 1,09 and 12,4 mM +/- 0,42 of acetate and sulfide respectively, were obtained with this co-culture grown on glucose in the presence of sulfate as terminal electron acceptor. This suggests that phosphogypsum at 15g/L has not an inhibitory effect despite containing significant amounts of heavy metals and possibly of other toxic products.

### III- Bioprecipitation of Zn containing in the phosphogypsum by the co-culture

Zn is one of the major heavy metals which is contained in the Tunisian phosphogypsum. The production of sulfide by the co-culture growth was at the origin of the precipitation of Zn containing in the culture medium with 15 g/l of phosphogypsum. This precipitation was studies after three weeks of incubation in the presence of glucose and fructose as carbon source.

Experiments were performed with the co-culture in the presence of 20 mM glucose or 20 mM fructose and 15g/l of phosphogypsum as sulfate source. They demonstrate a highly significant decrease in Zn concentrations after seven weeks of incubation at 37°C.

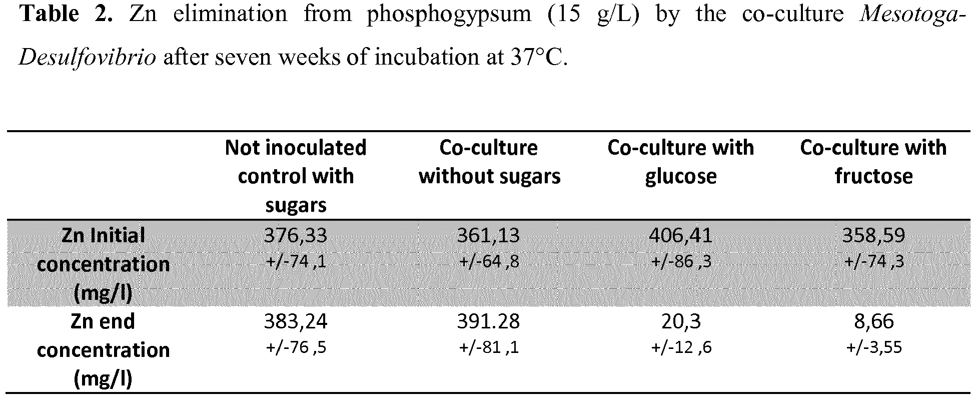

**Table 2** shows the variation of Zn concentrations at initial stage and after treatment with the co-culture. In the presence of glucose, the Zn concentration decreases from 406 mg/l +/- 86,3 to 20 mg/l +/- 12,6. The reduction of Zn concentration was more important when the co-culture used fructose as carbon/energy source. Indeed in this case the Zn concentration decreases from 358 mg/l +/- 74,3 to 8,66 mg/l +/- 3,55.

No significant Zn concentration decrease was observed under the same culture conditions by pure cultures of *Mesotoga prima* strain PhosAc3 or *D. vulgaris.*

The capacity of the sulfide-producing bacteria coupled to *Mesotoga* to catalyze the formation of insoluble precipitates mineral containing heavy metals, can represent an interesting process to immobilize and confine toxic metals.

In this respect, the sulfate reductive activity of *D. vulgaris* linked to the oxidation of sugars by *Mesotoga prima* strain PhosAc3 is not only beneficial for sulfide production but also for Zn removal. This is in agreement with the sulfate-reducing bacteria which have the capacity to catalyze the formation of insoluble precipitates mineral containing heavy metals, since sulfide produced by sulfate-reducing bacteria may be recycled to be used in phosphoric acid production. Therefore, the proposed co-culture appears as a good candidate to immobilize and confine toxic metals originating from phosphogypsum.

## Claims

1. Process for the co-culture of an anaerobic mesophilic bacterium of the *Mesotoga* lineage with at least one hydrogenotrophic sulfate-reducing bacterium.

2. Process for the co-culture according to claim 1, wherein the said co-culture is performed in a culture medium comprising at least one carbon source and at least one sulfur source.

3. Process for the co-culture according to claim 2, wherein the said at least one sulfur source is a sulfate or elemental sulfur and wherein the said at least one carbon source is a sugar.

4. Process for the co-culture according to any one of claims 2 to 3, wherein the said at least one sulfur source is phosphogypsum containing sulfate.

5. Process for the co-culture according to any one of claims 1 to 4, wherein the said anaerobic mesophilic bacterium of the *Mesotoga* lineage is the bacterial strain deposited at Collection Nationale de Cultures de Microorganismes (CNCM) under n°I-4238, or mutant thereof.

6. Process for the co-culture according to any one of claims 1 to 5, wherein the said hydrogenotrophic sulfate-reducing bacterium is chosen among the *Deltaproteobacteria* class, particularly among the *Desulfovibrionales* order, particularly among the *Desulfovibrio* genus, the *Desulfomicrobium* genus and the *Desulfocurvus* genus, and more particularly the said hydrogenotrophic sulfate-reducing bacterium is the species *Desulfovibrio vulgaris* of the *Desulfovibrio* genus, particularly the subspecies *Desulfovibrio vulgaris vulgaris.*

7. Process for the co-culture according to any one of claims 1 to 5, wherein the said hydrogenotrophic sulfate-reducing bacterium is chosen among the *Clostridia* class, particularly among the *Clostridiales* order, particularly among the *Desulfotomaculum* genus and the *Desulfosporosinus* genus and more particularly the said hydrogenotrophic sulfate-reducing bacterium is the species *Desulfotomaculum ruminis* of the *Desulfotomaculum* genus.

8. Process for the co-culture according to any one of claims 2 to 7, wherein the said sugar is a C6 sugar, in particular fructose or glucose.

9. Process for the co-culture according to any one of claims 2 to 7, wherein the said sugar is a C5 sugar, in particular xylose.

10. Process for the co-culture according to any one of claims 2 to 7, wherein the said sugar is sucrose or lactose.

11. Process for the co-culture according to any one of claims 2 to 10, wherein the said sugar has a concentration from 2 mM to 50 mM, particularly from 10 mM to 20 mM.

12. Process for the co-culture according to any one of claims 2 to 11, wherein the said co-culture is carried out at a temperature ranging from 20 °C to 50 °C, in particular at the temperature of 37°C.

13. Process for the co-culture according to any one of claims 2 to 12, wherein the said sulfate is at a concentration ranging from 2 to 40 mM, particularly 20 mM.

14. Process for the co-culture according to any one of claims 1 to 13, wherein the anaerobic mesophilic bacterium of bacterial strain deposited at Collection Nationale de Cultures de Microorganismes (CNCM) under n°I-4238 is co-cultured with the hydrogenotrophic sulfate-reducing bacterium *Desulfovibrio vulgaris* or *Desulfotomaculum ruminis,* in a culture medium containing sulfate and glucose.

15. Method for the cleanup of sites/sediments contaminated by phosphogypsum, comprising treatment of the contaminated sites/sediments with a co-culture of anaerobic mesophilic bacterium of the *Mesotoga* lineage, with a hydrogenotrophic sulfate-reducing bacterium, in presence of at least one sugar.
